# EUROPEAN PATENT APPLICATION

(11) **EP 1 504 776 A1**
(43) Date of publication of application: **09.02.2005**
(21) Application number: 03723331.9
(22) Date of filing: 12.05.2003
(51) Int. Cl.: A61L 27/56, A61L 27/58

(54) **MEMBER FOR REGENERATING JOINT CARTILAGE AND PROCESS FOR PRODUCING THE SAME, METHOD OF REGENERATING JOINT CARTILAGE AND ARITFICIAL CARTILAGE FOR TRANSPLANTATION**

(30) Priority: 13.05.2002 JP 2002137202; 12.08.2002 JP 2002234337
(71) Applicant: Toshiba Ceramics Co., Ltd., Tokyo (JP); MMT CO., LTD, OSAKA-SHI, OSAKA (JP); Yoshikawa, Hideki, Toyonaka-shi, Osaka 560-0004 (JP)
(72) Inventor: YOSHIKAWA, Hideki, Toyonaka-shi, Osaka 560-0004 (JP); MYOI, Akira, Toyonaka-Shi, Osaka 560-0083 (JP); TAMAI, Noriyuki, Hannan-shi, Osaka 599-0202 (JP)
(74) Representative: HOFFMANN EITLE
(86) International application number: PCT/JP2003/005887
(87) International publication number: WO 2003/094987

(57) **Abstract**

A regeneration member which, under nearly natural surroundings, is integrated into adjacent, surrounding, existent articular cartilage under good conditions and which is capable of early regenerating articular cartilage having an original thickness under continuous conditions, and a production method thereof are provided. Also, a regeneration method and a cultivation method, of articular cartilage, in vivo and in vitro are provided. Furthermore, an artificial articular cartilage obtained by these methods is provided.

Using a member for articular cartilage regeneration having a hydroxyapatite porous element, having a number of pores distributed therein, substantially all of said pores being three-dimensionally communicated to each other through open portions, a porosity of from 50% to 90%, both inclusive, and an average pore diameter of from 100 µm to 600 µm, both inclusive, articular cartilage is regenerated and cultivated.

## Description

### Technical Field

The present invention relates to a member for articular cartilage regeneration and a production method thereof, a regenerating method of and a cultivating method of, articular cartilage, and artificial articular cartilage for transplantation. And more specifically, the present invention relates to a member capable of regenerating cartilage peculiar to an articular portion and a production method thereof, and a regenerating method of articular cartilage in vivo or in vitro, a cultivating method of articular cartilage, and artificial articular cartilage for transplantation using a porous ceramic element as a matrix.

### Background Art

A joint is a connection portion through which a bone is movably linked with another bone, and the surface (articular face) of a mutual bone end in this connection portion is covered with articular cartilage. The periostea of the mutual bone end form an articular capsule to integratedly cover the connection portion. Between the bones covered with this articular capsule, a space called cavitas articulare is formed, which is filled with a synovial fluid therein.

The articular cartilage formed on the aforementioned articular face is normally approximately 2 mm thick in the human knee joint. When the knee joint is damaged to an extent of about 1 to about 4 mm² due to an external injury, disease, or the like, regeneration is possible by natural healing. However, if the joint is injured to an extent of as much as 20 mm², regeneration on its own is difficult, and also generally accompanied by great pain.

Furthermore, complete loss of articular cartilage because of various factors such as a tumor, necrosis, and the like leads to treatment such as, for example, implantation of an artificial joint in the site.

However, an artificial joint is artificially constructed like the joint function to the end, and is foreign matter to the living body, and thus making it difficult to maintain conformity to the living body.

Also, an artificial joint is required to be subjected to complicated movements under severe conditions in vivo, and so it is difficult to keep it for 20 years or more. Because of degradation, abrasion powders and the like of a resin, metal, or the like to be used as material thereof, lowering of the function and pain take place in some cases and also the durability may be insufficient.

Accordingly, a technique of regenerating articular cartilage itself in place of artificial articular treatment is desired.

In addition, in treatment of cartilage forming insufficiency, in which cartilage itself is difficult to form, as well, a technique of regenerating it as a living tissue is desired without depending on an artificial material as articular cartilage.

Although several proposals are presented for the technique of regenerating cartilage, these are each a level of cartilage present in a pre-stage in the ossification step of normal bone formation being studded in clearances of bones.

For instance, Japanese Unexamined Patent Application Publication No. 7-88174 discloses that a temporary bone-like bone protrusion cartilage bearing bone/cartilage having continuous bone film is formed by means of a graft using atherocollagen.

However, in the aforementioned graft, as noted like a temporary bone-like bone protrusion, the amount of cartilage is not sufficient; the graft does not have thickness and an amount as a joint cartilage having practical continuity.

As described above, articular cartilage is difficult to regenerate, and conventionally examples in which continuous film-like or layer-like cartilage is regenerated are rarely present. For the purpose of practical use, a technique capable of continuously obtaining such cartilage in a sufficient amount needs to be developed.

Also, in a recent presentation of study, it has been presented that articular cartilage is regenerated by perforating the articular surface and placing collagen containing bone morphogenetic protein (BMP) in a desired site.

However, the regenerated articular cartilage is not continuously formed with the neighboring, existent articular cartilage, and thus it cannot say to be perfect regeneration.

Furthermore, collagen, due to bovine spongiform encephalopathy (BSE), or other problems, application to the living body is likely to be avoided.

Hence, it is desirable to regenerate perfect articular cartilage in a continuous state without a border between regenerated and existent portions.

Japanese Patent No. 2951342 discloses that glass-like cartilage can be regenerated by employing an artificial prosthetic material, the implant portion of which is a closely packed portion of calcium phosphate-based ceramics comprising two-phase structure of a porous portion and a closely packed portion.

However, according to the aforementioned artificial prosthetic material, complete regeneration is said to take about 24 weeks even to an extent of several millimeters, and therefore it can be hardly said to be of early regeneration.

The present invention has been made to solve the above-described technical problems, and the object thereof is to provide a regeneration member which, under nearly natural surroundings, is integrated into adjacent, surrounding, existent articular cartilage under good conditions and which is capable of early regenerating articular cartilage having an original thickness under continuous conditions, and a production method thereof.

Also, other objects of the present invention are to provide a regeneration method of and a cultivation method of, articular cartilage, and further to provide artificial articular cartilage for implantation obtained by methods thereof.

### Disclosure of the Invention

A member for articular cartilage regeneration relating to the present invention is characterized in that the member comprises a hydroxyapatite porous element having a number of pores distributed therein, substantially all of said pores being three-dimensionally communicated to each other through open portions, a porosity of from 50% to 90%, both inclusive, and an average pore diameter of from 100 µm to 600 µm, both inclusive.

The term "regeneration" herein includes "to newly form" in meaning.

A porous element of hydroxyapatite having the porosity and porous shapes as mentioned above, in which mesenchymal cells, mesenchymal stem cells, bone marrow cells and the like needed for forming articular cartilage tend to enter from the bone inside and fix, allows articular cartilage to regenerate early.

The pores of the above-mentioned porous element are formed by agitation foaming, and preferably ones in which almost sphere-like, adjacent pores are opened to each other in contact portions to form continuous sphere-like open pores.

Also, the average diameter of the open portion of each pore of the aforementioned porous element is preferably 20 µm or more.

Alternatively, a member for articular cartilage regeneration relating to the present invention comprises a ceramic porous element; and a living body-absorbing member; said ceramic porous element being formed with pores, substantially all of said pores cooperating to form three-dimensionally communicated continuous sphere-like open pores byway of openings thereof to such an extent that, of said pores, ones with a pore diameter of 5µm or more occupying 85% or more of the total pore volume in terms of a pore diameter distribution determined by mercury porosimeter measurement, said living body-absorbing member being carried on a pore inner surface of said ceramic porous element and containing a bone morphogenetic inductive factor therewithin.

The aforementioned bone morphogenetic inductive factor is preferably any one selecting from the group consisting of bone morphogentic protein (BMP) , transforming growth factor β (TGF-β), an osteoinductive factor (OIF), an insulin-like derived growth factor (IGF), a platlet derived growth factor (PDGF), and a fibroblast growth factor (FGF).

To apply the above-described member for articular cartilage regeneration to the human body, the above-mentioned bone morphogenetic inductive factor is particularly preferably recombinant human bone morphogentic protein (rhBMP).

The above-mentioned bone morphogenetic inductive factor is preferably homogeneously intermingled in a living body-absorbing member.

In addition, the pore of the aforementioned porous ceramic element is formed by agitation foaming and preferably has a porosity of 50% to 90%, both inclusive, an average pore diameter of 100 µm to 600 µm, both inclusive, and an average diameter of the open portion of each pore in the above-mentioned sphere-like open pores being 20 µm, or more.

Furthermore, the above-mentioned ceramic porous element preferably comprises at least one kind selecting from the group consisting of alumina, zirconia, silica, mullite, diopside, wollastnite, alite, belite, akermanite, monticellite, glass for the living body, and calcium phosphate-based ceramics.

Of these, calcium phosphate-based ceramics is preferable, and examples of this calcium phosphate-based ceramics include hydroxyapatite, tricalcium phosphate, apatite fluoride In the present invention, particularly, hydroxyapatite is preferable for use.

Also, the aforementioned living body-absorbing member preferably has the gradual release properties of a bone morphogenetic inductive factor. Materials having such characteristic for suitable use include organic compounds, e.g., a polymer of lactic acid and/or glycolic acid, a block copolymer of a polymer of lactic acid and/or glycolic acid and polyethylene glycol, a copolymer of lactic acid and/or glycolic acid, p-dioxanone and polyethylene glycol, and atherocollagen.

Even of these, in particular, a block copolymer (PLA-PEG) is preferable that has polylactic acid having a number average molecular weight of 400 to 1000000 and polyethylene glycol in which the molar ratio of the polylactic acid to the polyethylene glycol ranges from 25:75 to 75:25.

Moreover, a copolymer (PLA-DX-PEG) of lactic acid and/or glycolic acid, p-dioxanone, and polyethylene glycol can also similarly suitably be used.

A producing method of a member for articular cartilage regeneration relating to the present invention is characterized by comprising a step of adding a living body-absorbing material to a solvent or a dispersing medium and then blending a bone morphogenetic inductive factor therewith to prepare a mixture solution; a step of infiltrating the aforementioned mixture solution into a ceramic porous element in which the pores are formed by agitation foaming and which forms a continuous, sphere-like, open pore produced by three-dimensionally communicating each pore to each other through open portions; and a step of removing the solvent or the dispersing medium in the above-mentioned porous ceramic element and carrying a living body-absorbing member and a bone morphogenetic inductive factor to obtain the member for articular cartilage regeneration.

In the step of preparing the aforementioned mixture solution, when the living body-absorbing member comprises an organic compound, acetone is preferably used as the solvent or the dispersing medium.

A regenerating method of articular cartilage relating to the present invention is characterized by embeding a porous element in the site deeper than the under surface of the articular cartilage layer of the articular face.

According to the above-described method, bone cells are incorporated into the inside of a porous element, a bone under cartilage is formed on the surface of the porous element, and further on the upper surface thereof articular cartilage can be regenerated with a thickness equivalent to an existent articular cartilage close to the surroundings.

In the regenerating method of the aforementioned articular cartilage, the porous element is preferably buried in such a way that at least a portion of the aforementioned porous element is made contact with mesenchymal cells, mesenchymal stem cells, or bone marrow cells, in a bone.

By embeding a porous element contacted with the aforementioned mesenchymal cells or the like, the regeneration of articular cartilage can be promoted.

Also, the porous element is preferably buried such that the upper surface (the end face of the articular surface side) of the aforementioned porous element is exposed to the articular face.

In order for articular cartilage not to be prevented from being reproduced at the original damaged site with an original thickness, as discussed above, a space in which articular cartilage to be regenerated is occupied is preferably provided.

Furthermore, after the aforementioned porous element is buried, at least a portion of the porous element is preferably made contact with the articular fluid.

Contacting the aforementioned porous element with the articular fluid promotes the regeneration of articular cartilage.

In addition, a regenerating method of articular cartilage relating to the present invention preferably involves cutting an articular capsule open to expose the articular face, making perforation in a desired position, embeding the aforementioned porous element in a site deeper than the under surface of the articular cartilage layer within the perforation, and subsequently suturing the aforementioned articular capsule.

As treated discussed above, putting the mechanism back to the original movable conditions enables the articular cartilage to be early regenerated.

Preferably, the aforementioned perforation is formed so that the lower end thereof reaches the proximity to the bone marrow.

Perforating to the depth close to the bone marrow allows sufficient bone marrow cells to be introduced into the porous element.

Additionally, when a growth articular cartilage layer is present, embeding at least a portion of the aforementioned porous element to reach the depth reaching the growth articular cartilage layer displays a tendency to promote the regeneration of articular cartilage even without perforating the hole to reach the proximity to the bone marrow.

The aforementioned porous element used in the present invention, the porosity of which is from 50% to 90%, both inclusive, preferably has as a whole a communicated pore capable of permeation and movement by cells.

According to a porous element having a porosity and a pore shape as indicated above, because mesenchymal cells, mesenchymal stem cells, bone marrow cells and the like are likely to permeate from the bone inside and fix, it is possible to early regenerate articular cartilage.

Also, the aforementioned porous element is preferably made up of hydroxyapatite.

Hydroxyapatite is the primary component of a bone, is permitted to apply to the human body, and is an appropriate material from the viewpoint of assimilation to a bone, adhesion properties, strength, early recovery and the like. Moreover, it is suitable as a scaffold for cells.

Furthermore, as the aforementioned porous element, a material in which the pores are formed by agitation foaming can be suitably used.

A porous element in which the pores are formed by agitation foaming has almost sphere-like pores and dense frame (for example, the porosity of the frame of the porous element per se is not more than 5%), thus obtaining a high strength despite the porosity of from 50 % to 90 % relative to the whole volume of the porous element inclusive of the pores. In addition, said porous element has capillary properties in which mesenchymal cells, blood constituents and the like readily penetrate. In addition, the surface area per volume is large, and tends to have suitable properties as a scaffold of permeation cells. Further, said agitation foaming has an advantage that it is easy to produce a porous element having uniform and even size pores.

Also, a regenerating method of articular cartilage relating to the present invention is characterized by placing on the articular face a living body-absorbing member of a porous element, which contains a bone morphogenetic inductive factor, and which has gradual release properties thereof, and fixing.

Moreover, the aforementioned member preferably renders at least a portion thereof to contact with the articular fluid.

Furthermore, a ceramic porous element carrying on the pore inner surface thereof a living body-absorbing member and a bone morphogenetic inductive factor is made buried in the articular face, and then articular cartilage can also be regenerated or formed by fixing the articular cartilage on the articular face.

During this procedure, after the aforementioned ceramic porous element is buried in the articular face, at least a portion of the ceramic porous element is preferably made contact with the articular fluid.

For the aforementioned ceramic porous element carrying on the pore inner surface thereof a living body-absorbing member and a bone morphogenetic inductive factor, used in the regenerating method of the aforementioned articular cartilage, the member for articular cartilage regeneration relating to the present invention as described above can suitably be used.

Also, the aforementioned articular cartilage is preferably made grown in such a way that the articular cartilage has a homogeneous thickness of 400 µm or more.

In addition, a cultivating method of articular cartilage relating to the present invention is characterized by placing a living body-absorbing member of a porous element, which contains a bone morphogenetic inductive factor, and which has gradual release properties thereof, close to or near cells to be possibly articular cartilage, and contacting at least a portion of the aforementioned member with the articular fluid.

Additionally, a ceramic porous element carrying on the pore inner surface thereof a living body-absorbing member and a bone morphogenetic inductive factor is placed close to or near cells to be possibly articular cartilage, or cells to be possibly articular cartilage are introduced in the pores of the ceramic porous element, and at least a portion of the aforementioned ceramic porous element is made contacted with the articular fluid, thereby being capable of cultivating articular cartilage as well.

Here, a cell to be possibly the aforementioned articular cartilage preferably comprises a mesenchymal stem cell.

Furthermore, on at least a portion of the pore inner surface of a ceramic porous element carrying on the pore inner surface thereof a living body-absorbing member and a bone morphogenetic inductive factor is formed a bone, and then further on at least a portion of the surface of the ceramic porous element, articular cartilage can also be cultivated by means of the above-described cultivation method.

In the above-described cultivation method, as the ceramic porous element carrying on the pore inner surface thereof a living body-absorbing member and a bone morphogenetic inductive factor was suitably used themember for articular cartilage regeneration relating to the present invention as noted above, and also the aforementioned articular cartilage is preferably made cultivated in such a way that the articular cartilage has a homogeneous thickness of 400 µm or more.

Moreover, an artificial articular cartilage for implantation relating to the present invention is characterized in that on at least a portion of the pore inner surface of a ceramic porous element carrying on the pore inner surface thereof a living body-absorbing member and a bone morphogenetic inductive factor, articular cartilage is formed.

In the aforementioned artificial articular cartilage for implantation, the articular cartilage preferably has a homogeneous thickness of 400 µm or more.

Also, preferably, in the aforementioned artificial articular cartilage for implantation, on at least a portion of the surface of a bone formed by fixation of the bone cells in the pore inside of the aforementioned ceramic porous element is formed an articular cartilage layer, or on at least a portion of the surface of a bone formed by fixation of the bone cells in the pore inside of the aforementioned ceramic porous element is formed an articular cartilage layer not containing a ceramic porous element and further on at least a portion of the surface of the bone cell layer is formed an articular cartilage layer.

Here, as the aforementioned ceramic porous element carrying on the pore inner surface thereof a living body-absorbing member and a bone morphogenetic inductive factor, the member for articular cartilage regeneration relating to the present invention as noted above can suitably be used.

Furthermore, a regenerating method of or a cultivating method of articular cartilage relating to the present invention is characterized in that close to or near the articular cartilage collected from articular cartilage within the living body or from the living body is gradually released a bone morphogenetic inductive factor.

During this procedure, the aforementioned bone morphogenetic inductive factor is preferably gradually released in the presence of an articular fluid.

### Brief Description of the Drawings

Fig. 1 shows a microscope photograph of the buried portion of a member for articular cartilage regeneration after 6 weeks in Example 1.
Fig. 2 shows a portion of an enlarged photograph taken along the line A-A' in Fig. 1, and Fig. 3 shows an enlarged photograph of an upper portion layer in Fig. 2.
Fig. 4 shows a microscope photograph of the buried portion of a member for articular cartilage regeneration after 6 weeks in Comparative Example 1.
Fig. 5 shows an enlarged photograph near C' portion of Fig. 4, and Fig. 6 shows an enlarged photograph of an upper portion of Fig. 5.
Figs. 7 and 8 show microscope photographs of a ceramic porous element of hydroxyapatite relating to the present invention; Fig. 7 shows a view having a magnification of 150 times and Fig. 8 shows a view having a magnification of 10,000 times.
Fig. 9 shows the pore distributions of the ceramic porous element indicated in Figs. 7 and 8 by means of a mercury porosimeter.
Fig. 10 is a schematic diagram near articular cartilage of a femur in a knee joint of a rabbit.
Fig. 11 is a schematic diagram of an enlarged, buried portion of the porous element in Fig. 10.
Fig. 12 is a schematic diagram of an enlarged regeneration portion of articular cartilage.
Fig. 13 is a schematic diagram in the case where on the articular face of a femur in a knee joint of a rabbit, a perforation is made so that the lower portion thereof reaches the proximity of the bone marrow.

### Best Mode for Carrying Out the Invention

Hereinafter, the present invention will be set forth in detail partly in reference to drawings.

A member for articular cartilage regeneration relating to the present invention comprises a hydroxyapatite porous element having a number of pores distributed therein, substantially all of said pores being three-dimensionally communicated to each other through open portions, having a porosity of from 50% to 90%, both inclusive, and having an average pore diameter of from 100 µm to 600 µm, both inclusive.

According to a porous element having the porosity and porous shapes as mentioned above, because mesenchymal cells, mesenchymal stem cells, bone marrow cells and the like and blood and the like needed for forming articular cartilage tend to enter from the bone inside and fix, articular cartilage can be regenerated early.

In addition, the aforementioned average pore diameter can be determined using a method by resin embedding.

When the aforementioned porosity is less than 50%, the aforementioned mesenchymal cell and the like make it difficult to permeate the inside of a porous element, thereby rendering early regeneration of articular cartilage difficult.

On the other hand, if the aforementioned porosity exceeds 90%, the fixation of the aforementioned mesenchymal cell and the like is inferior, in this case as well, early regeneration of articular cartilage becomes difficult, and also sufficient strength is not obtainable.

The aforementioned porosity is more preferably from 65% to 85%, both inclusive.

Additionally, the above-mentioned porous element can be, as appropriate, used as a granular element as well.

Pores of the above-mentioned porous element are formed by agitation foaming, and preferably almost sphere-like, adjacent pores are opened to each other in contact portions to form continuous sphere-like open pores.

As discussed above, a porous element used in the present invention having as a whole a communicated pore capable of permeation and movement by cells is suitably employed.

Moreover, the average diameter of the open portion of each pore in the above-mentioned porous element is preferably 20 µm or more, more preferably 40 µm or more. The average diameter of this open portion can be determined with a mercury porosimeter (the mercury pressure process).

Such porous element is disclosed, for example, in Japanese Unexamined Patent Application Publication No. 2000-302567.

Alternatively, a member for articular cartilage regeneration relating to the present invention comprises a ceramic porous element; and a living body-absorbing member; said ceramic porous element being formed with pores, substantially all of said pores cooperating to form three-dimensionally communicated continuous sphere-like open pores byway of openings thereof to such an extent that, of said pores, ones with a pore diameter of 5µm or more occupying 85% or more of the total pore volume in terms of a pore diameter distribution determined by mercury porosimeter measurement, said living body-absorbing member being carried on a pore inner surface of said ceramic porous element and containing a bone morphogenetic inductive factor therewithin.

As the aforementioned bone morphogenetic inductive factor, a variety of bone morphogenetic relating proteins, i.e., extraction components from bone tissues, can be used. Examples of the bone morphogenetic inductive factor include bone morphogentic protein (BMP) , transforming growth factor β (TGF-β), a cartilage-derived morphogenetic protein (CDMP), an osteoinductive factor (OIF), an insulin-like derived growth factor (IGF), a platlet derived growth factor (PDGF), a fibroblast growth factor (FGF) , a connective tissue growth factor (CTGF), a vasular endothelial growth factor (VEGF), a hepatocyte growth factor (HGF), a placenta-derived growth factor (PIGF), angiopoietin, a bisphosphonate, a mevalonic acid path inhibitor, a signal transfer actuating agent, and the like.

Of these bone morphogenetic inductive factors, the use of one species or two species or more is preferable that are selected from the group consisting of bone morphogentic protein (BMP), transforming growth factor β (TGF-β), a cartilage-derived morphogenetic protein (CDMP), an osteoinductive factor (OIF), an insulin-like derived growth factor (IGF), a platlet derived growth factor (PDGF), and a fibroblast growth factor (FGF), and further, to apply the aforementioned member for bone formation to the human body, a human bone morphogentic protein (hBMP) substantially not containing the other proteins derived from human is more preferable.

In particular, from the standpoint of being capable of obtaining a large amount of material that is clinically stable in immunity and the like and has stable quality, a recombinant human bone morphogentic protein (rhBMP) obtained by the gene recombinant technique is preferable. In other words, a cell containing recombinant DNA bearing a base sequence encoding a human osteoinductive factor or a transformant of a microorganism or the like is cultivated, and a rhBMP produced by a transformant thereof is isolated and purified, whereby obtaining the material.

These rhBMPs include, for example, rhBMP-2, rhBMP-3, rhBMP-4 (also called rhBMP-2B), rhBMP-5 or rhBMP-6, rhBMP-7rhBMP-8, a heterodimer of a rhBMP, or transformants thereof and partially lost species thereof. These can be used singly or as a mixture of two species or more. Of these, for cartilage regeneration, rhBMP-2 or rhBMP-7, which has a large effect, is preferable, and particularly rhBMP-2 is preferable.

In order for the above-described bone morphogenetic inductive factor to develop, a matrix carrying it is necessary. As a matrix thereof, in the present invention, a ceramic porous element is used, having a number of pores distributed therein, substantially all of said pores being three-dimensionally communicated to each other through open portions to form continuous sphere-like open pores, which has the pore volume having a pore diameter of 5 µm or more and having a volume of 85% or more relative to the total pore volume in the pore diameter distribution determined by means of a mercury porosimeter.

Because of such structure of the matrix, the skeletal portion is close, has sufficient strength as a whole, and can stably support the living body-absorbing member for a long period of time. Also, a pore itself is averagely large, and thus through the large open portion of the aforementioned pore cells and body fluids can swiftly and efficiently transfer and circulate.

Moreover, in the aforementioned matrix, each pore shape is almost a sphere, and even though the porosity thereof is large, the shape thereof is kept without loss. Thus, the surface area inside the pore is large and the matrix can carry a living body-absorbing member and a bone morphogenetic inductive factor on the surface area inside the pore in a high density.

The aforementioned bone morphogenetic inductive factors, in terms of carrying uniform articular cartilage formation as the entire member, are preferably homogeneously intermingled in the living body-absorbing member.

For the aforementioned ceramic porous element as well, from the viewpoint of ready introduction of cells and nutrients from the bone inside to cartilage formation portions and ready entire reach thereof and the like, the porosity is preferably from 50% to 90%, both inclusive, more preferably from 65% to 85%, both inclusive. Also, the average pore diameter is preferably from 100 µm to 600 µm, both inclusive.

In addition, the average diameter of the open portion of the aforementioned ceramic porous element is preferably 20 µm or more, more preferably 40 µm or more.

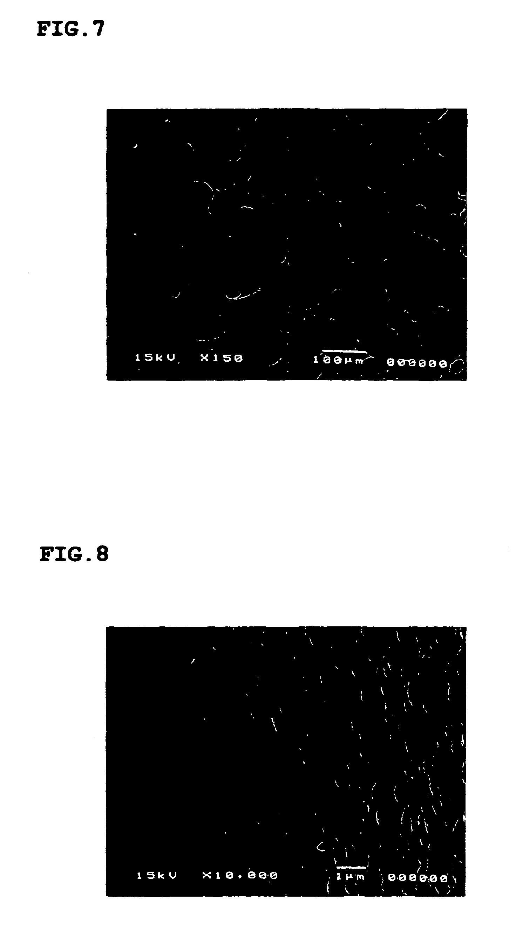

Figs. 7 and 8 show microscope photographs of a ceramic porous element made up of hydroxyapatite relating to the present invention; Fig. 7 shows a view having a magnification of 150 times and Fig. 8 shows a view having a magnification of 10,000 times.

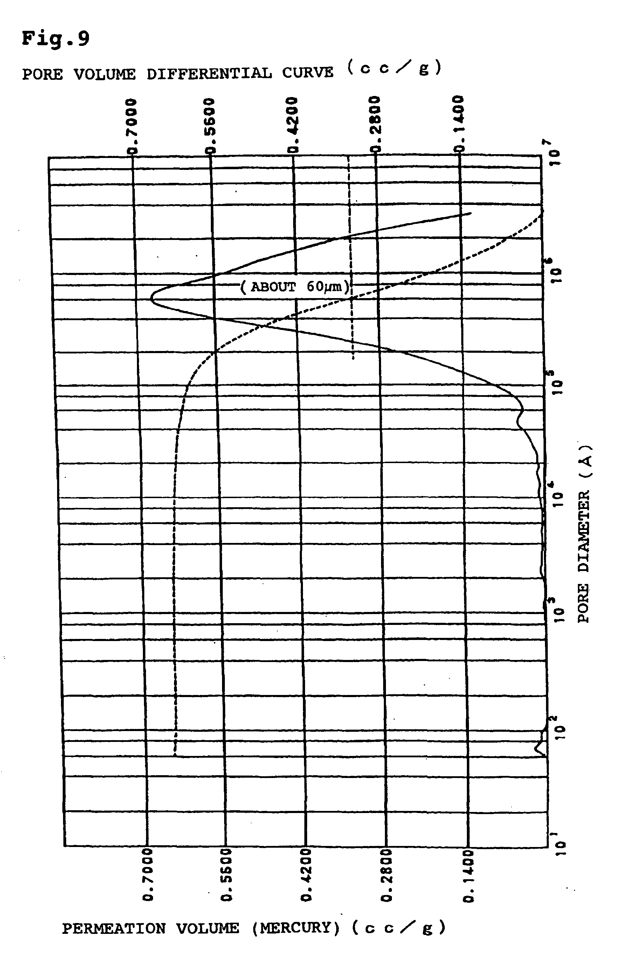

Additionally, Fig. 9 shows the pore distributions of the ceramic porous element of the aforementioned hydroxyapatite determined by means of a mercury porosimeter (the mercury pressure process).

A ceramic porous element having the aforementioned pores can be readily produced by agitation foaming. A ceramic porous element formed by agitation foaming, in which a pore is close in skeleton and is almost a sphere shape, so that high strength is obtainable, is preferable.

Specifically, for example, by means of a production method as described below, a ceramic porous element as noted above can be obtained. An example for using hydroxyapatite as the material will be set forth.

First, to hydroxyapatite powder is added polyethylene-imine or the like as the crosslinkage polymerizing resin, and then using water as a dispersing medium the resulting material is blended and shredded to prepare a slurry.

Then, to this slurry was added polyoxyethylene lauryl ether or the like as the foaming agent to agitation foam.

Furthermore, sorbitol glycidyl ether or the like is added as the crosslinking agent, and the resultant foam-like slurry is cast. After the slurry is dried while keeping the foam structure, it is sintered at about 1100 to about 1300°C to obtain a porous element of hydroxyapatite.

Preferably, the aforementioned ceramic porous element does not have living body damaging properties and is ceramics that is a material having sufficient mechanical strength, and specifically comprises at least one kind selecting from the group consisting of alumina, zirconia, silica, mullite, deopside, wollastnite, alite, belite, arkelmanite, monticellite, glass for the living body, and calcium phosphate-based ceramics.

Of these, because of being excellent in living body conformity, calcium phosphate-based ceramics is preferable. Examples of this calcium phosphate-based ceramics include hydroxyapatite, tricalciumphosphate, apatite fluoride. In the present invention, particularly, from the viewpoint of assimilation to a bone, adhesion properties, strength, and the like, the ceramics preferably comprises hydroxyapatite, which is the main constituent of a bone.

In addition, to gradually release the above-described bone morphogenetic inductive factor, within an appropriate period for regenerating articular cartilage of from several weeks to scores of weeks, a body-absorbing member to be combined with the above-mentioned bone morphogenetic inductive factor is essential. When a bone morphogenetic inductive factor is directly applied to a portion in which articular cartilage is to be formed, the bone morphogenetic inductive factor immediately flows, so that articular cartilage is not formed.

Hence, preferably, a living body-absorbing material to be combined with a bone morphogenetic inductive factor exhibits gradual release properties of the bone morphogenetic inductive factor, does not have living body damaging properties, and is gradually absorbed into cells in the living body, and at the same time gradually releases the bone morphogenetic inductive factor.

Materials having such characteristics allows inorganic materials such as tricalcium phosphate, but from the viewpoint of ease of controlling the time required for living body absorption, and the like, an organic compound is preferable. With an organic compound, the gradual release period can be adjusted by controlling the molecular weight thereof, and further a bone morphogenetic inductive factor can be distributed to the pore insides of a ceramic porous element of a matrix homogeneously and in a wide range.

Also, in terms of removing infection due to bacteria or the like, the use of an organic compound obtained by synthesis is more preferable.

As the aforementioned living body-absorbing material, specifically, a polymer material comprising both hydrophobic and hydrophilic properties is suitably used. Examples of the living body-absorbing material include a polymer of lactic acid and/or glycolic acid, a block copolymer of a polymer of lactic acid and/or glycolic acid and polyethylene glycol, a copolymer of lactic acid and/or glycolic acid, p-dioxanone, and polyethylene glycol (PLA-DX-PEG), atherocollagen, and the like.

Of these, in particular, from the standpoint of the absorption rate into cells in the living body and the release rate of a bone morphogenetic inductive factor, adhesion properties to the aforementioned ceramic porous element, elasticity, and the like, polylactic acid/polyethylene glycol copolymer (PLA-PEG) is preferable, and further the number average molecular weight of polylactic acid (PLA) is from 400 to 1000000, both inclusive, more preferably from 400 to 5000, both inclusive. Additionally, the molar ratio of the aforementioned polylactic acid (PLA) to polyethylene glycol (PEG) is preferably from 25:75 to 75:25. In particular, the number average molecular weight as a whole is from 9000 to 9700, and the molar ratio is more preferably from 60:40 to 70:30.

Moreover, as required, a ceramic component, or the like similar to the material of the above-mentioned ceramic porous element may be added.

Similarly, a copolymer of lactic acid and/or glycolic acid, p-dioxanone, and polyethylene glycol (PLA-DX-PEG) having a number average molecular weight from 5000 to 20000, both inclusive, and preferably from 8000 to 10000, both inclusive, is suitable as a living body-absorbing member as well. The number average molecular weight thereof is more preferably from 8900 to 9400, both inclusive.

For this PLA-DX-PEG, the ratio of lactic acid and/or glycolic acid to p-dioxanone to polyethylene glycol is preferably 26 to 60:4 to 25:25 to 70 in a molar ratio.

A specific producing method of this PLA-DX-PEG can use, such as, a method described in Japanese Unexamined Patent Application Publication No. 2000-237297.

A member for articular cartilage regeneration as noted above can be obtained by a production method of that comprises a step of adding a living body-absorbing material to a solvent or a dispersing medium and then blending a bone morphogenetic inductive factor therewith to prepare a mixture solution, a step of infiltrating the aforementioned mixture solution into a ceramic porous element in which pores are formed by agitation foaming and which forms a continuous sphere-like open pore produced by three-dimensionally communicating each pore to each other through open portions, and a step of removing the solvent or the dispersing medium in the above-mentioned porous ceramic element and carrying a living body-absorbing member and a bone morphogenetic inductive factor to obtain the member for articular cartilage regeneration.

In addition, in the step of preparation of the aforementioned mixture solution, as a solvent or a dispersing medium, acetone, methylene chloride, chloroform, ethanol, or the like can be preferably used, particularly, acetone is preferably used. These solvents or dispersing media play a role in homogeneously carrying a living body-absorbing member and a bone morphogenetic inductive factor on the inner surface of the pore of a ceramic porous element, and subsequently can be removed by evaporation, freeze drying, reduction pressure drying, or the like, and preferably do not remain within the resultant member for articular cartilage regeneration.

Now, a regenerating method of articular cartilage relating to the present invention will be set forth.

A regenerating method of articular cartilage relating to the present invention is characterized by embeding a porous element in the site deeper than the under surface of the articular cartilage layer of the articular face.

The above-described method involves incorporating bone cells from the bone inside into the inside of a porous element, further forming a bone layer not containing a porous element called a cartilage inferior bone on the surface of the porous element, and subsequently regenerating articular cartilage on the upper surface thereof, with a thickness of the cartilage equivalent to an existent articular cartilage close to the surroundings.

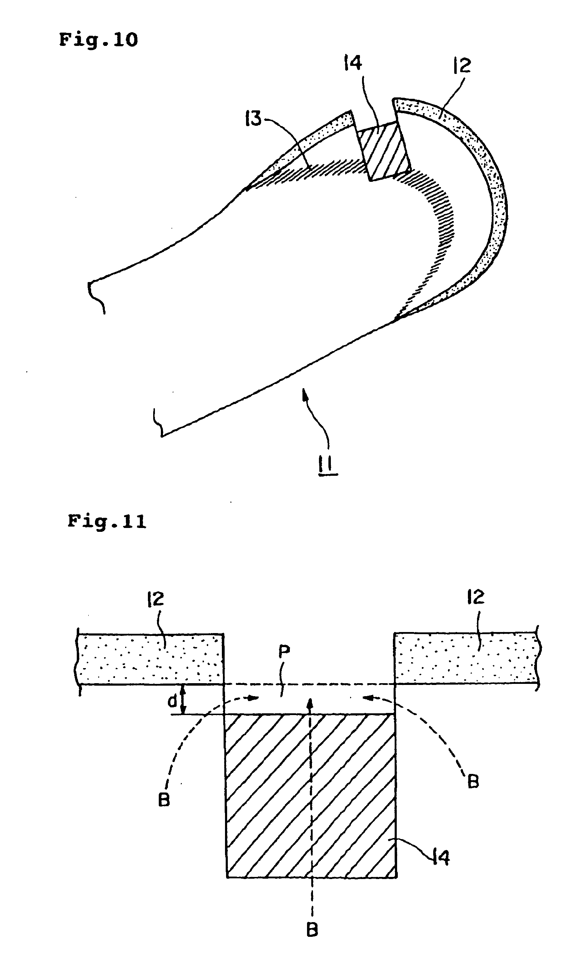

A regenerating method of articular cartilage relating to the present invention, first, involves cutting an articular capsule open to expose the articular face. For instance, Fig. 10 is a schematic diagram in the case where articular cartilage of a knee joint is regenerated.

As shown in Fig. 10, in the knee joint of a femur 11, in a desired site required for regeneration of articular cartilage 12, the bone is ground to perforate to the depth direction from the articular face. Then, in the perforation, a porous element 14 having a shape almost equivalent to the perforation is buried in a position deeper than the under surface of the layer of the articular cartilage 12.

In the aforementioned porous element 14, as in Fig. 10, at least a part thereof is buries until a position close to a portion in which many mesenchymal cells, mesenchymal stem cells, bone marrow cells, and the like are present.

Embeding the porous element 14 in contact with mesenchymal cells, mesenchymal stem cells, bone marrow cells, and the like allows the regeneration of articular cartilage to be promoted.

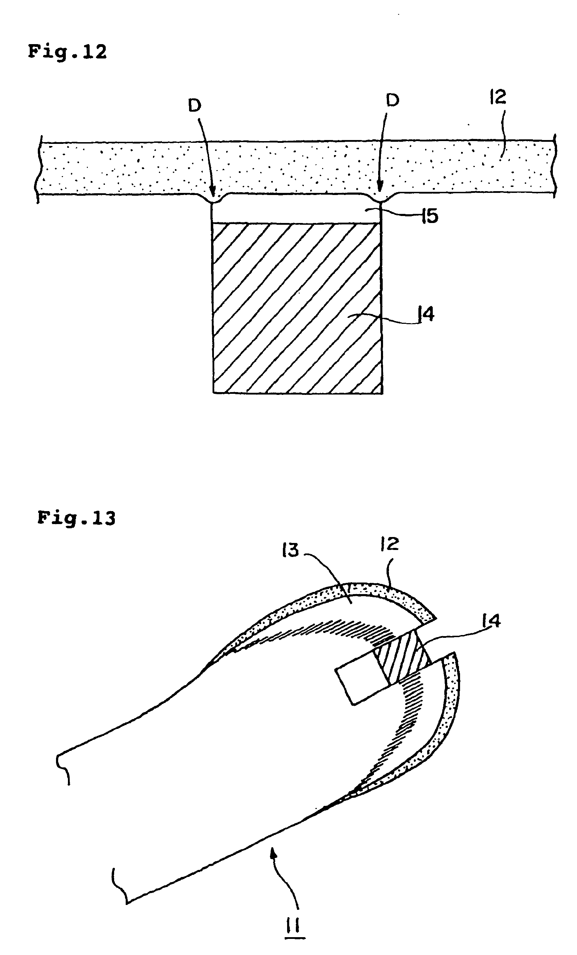

In this procedure, more preferably, the aforementioned perforation, as indicated in Fig. 13, is formed deeply until a site in which many bone marrow cells are present and the lower end thereof is made to reach the proximity of the bone marrow.

Perforation until a position of a depth near a bone marrow can introduce sufficient bone marrow cells into the inside of the porous element 14.

Additionally, a deep portion of such deep perforation may be buried with a porous, granular element, or the like, or may make left hollow.

When a growth cartilage layer 13 is present, at least a portion of the aforementioned porous element 14 is preferably buried until a position of a depth reaching the growth cartilage layer 13.

In general, although the growth cartilage layer 13 disappears with growth or aging, when a growth cartilage layer 13 is present, as noted above, it is confirmed that embeding the porous element 14 until a position close thereto is easy to provide an excellent regeneration effect of articular cartilage as compared with the case where the porous element is not in contact with the growth cartilage layer 13.

Also, even though with the thickness of the aforementioned porous element 14, a thinner case decreases damage given to a bone because a small depth of a perforation by bone grinding is sufficient, from the viewpoint of ease of permeation by cells and the like from the bone inside, or the like, a thickness roughly capable of sufficiently fixing the porous element within the aforementioned perforation is preferable.

In addition, according to a method relating to the present invention, since articular cartilage is recovered in the original, damaged position with the original thickness, a porous element is preferably buried so that an obstacle is absent in a portion to cause articular cartilage to be regenerated, that is, the upper surface of the porous element 14 is in a state of exposition.

Fig. 11 is an enlarged diagram of the site where the porous element 14 in Fig. 10 is made buried.

As shown in Fig. 11, the porous element 14 is buried and fixed in a position d deeper than the under surface of the layer of the existent articular cartilage 12, i.e., in a state of forming the depression portion P. Then, on the upper surface of the porous element 14, an articular capsule is sutured in a state of exposing the articular face, and the treatment is completed.

After the completion of treatment, as time elapses, bone-contained mesenchymal cells, mesenchymal stem cells, bone marrow cells and the like and blood and the like penetrate from the bone inside into the porous element 14, or are provided from the side portion of the depression portion P, that is, from the direction indicated by the arrow B, to the aforementioned depression portion P.

Subsequently, bone cells permeate the inside of the porous element 14, as in a state shown in Fig. 12, articular cartilage is regenerated.

In other words, in the portion in which the porous element 14 is buried, with containing the porous element 14, bone is formed therein. Also, at the same time, on the upper surface of the porous element 14, as described in Fig. 12, a new bone layer called a cartilage inferior bone 15 is formed.

Further, on the upper surface of the aforementioned cartilage inferior bone 15, the articular cartilage 12 is formed. Articular cartilage to be regenerated is formed, the thickness of which is equivalent to adjacent, surrounding, existent articular cartilage, to be integrated in a good condition. Moreover, the border portion of the surface is continuously formed in smooth conditions also without cracks, damage, projections and injections, and the like.

On the other hand, where the upper surface of the porous element 14 is equivalent to the depth position of the under surface of the layer of the existent articular cartilage 12, or is shallowly buried, in other words, where the upper surface is buried with a state of not forming the depression portion P, articular cartilage is not formed, also, articular inferior bone is not formed as well.

In addition, the under surface D of the articular cartilage of the border portion is slightly easy to be thick as compared with the other portions of the articular cartilage layer.

Depending on the depth and position of the upper surface of the porous element 14 buried, a formation state of the under surface D of the articular cartilage of the aforementioned border portion varies, and thus a supply state of mesenchymal cells and the like from the bone inside is thought to be a cause.

As discussed above, because embeding the porous element 14 in a position d deeper than the under surface of the layer of the existent articular cartilage 12, i.e., in a state of forming the depression portion P causes mesenchymal cells and the like to be supplied from the side portion of the depression portion P, and also since a natural healing power of early recovering the damaged portion is acted, on the under surface D of the articular cartilage of the border portion, articular cartilage is estimated to be thick.

In the treatment noted above, after the porous element is buried and the articular capsule is sutured, an articular fluid is preferably filled within the articular capsule.

The aforementioned porous element, because at least a portion thereof makes contact with the articular fluid, receives any stimulation, thereby being considered to render the regeneration of articular capsule to be promoted.

In addition, with the joint being movable, the change of a load, and stimulation such as the pressure change of the articular fluid are preferably provided. Providing such stimulation is thought to contribute to the promotion of articular cartilage regeneration.

For the aforementioned porous element, the porosity is from 50% to 90%, both inclusive, and as a whole preferably has continuous, communicated pores capable of permeation and movement of cells.

As long as the aforementioned ceramic porous element does not have living body damaging properties, and also is a material having sufficient mechanical strength, any inorganic materials, organic materials, or composites of inorganic and organic compounds are allowable. Moreover, a living body-absorbing material is permitted as well.

Examples of the ceramic porous element for suitable use include titanium, alumina, zirconia, silica, mullite, deopside, wollastnite, alite, belite, arkelmanite, monticellite, glass for the living body, and calcium phosphate-based ceramics, a polymer or a copolymer of lactic acid and/or glycolic acid, collagen, and the like.

These materials may be used in a combination of two or more materials.

Of these, calcium phosphate-based ceramics, which is excellent in living body conformity and already permitted to applications to the human body, is preferable and examples of the ceramics for suitable use include hydroxyapatite, tricalcium phosphate, apatite fluoride, and the like.

In the present invention, particularly, from the viewpoint of assimilation to a bone, adhesion properties, strength, early recovery and the like, the ceramics preferably comprises hydroxyapatite, which is the main constituent of a bone.

When the regeneration of articular cartilage is carried out using a porous element comprising a composition like the above, the regeneration of articular cartilage is possible without using a special factor such as a bone morphogenetic inductive factor, but to still more surely regenerate, the aforementioned porous element is preferably used along with a bone morphogenetic inductive factor.

Furthermore, a regenerating method of articular cartilage preferably involves placing on the articular face, a living body-absorbing member of a porous element, which contains a bone morphogenetic inductive factor, and which has gradual release properties thereof, and fixing. For instance, as rendering a bone of a patient himself to be a scaffold, on the surface thereof can be formed articular cartilage.

When the aforementioned bone morphogenetic inductive factor is singly directly applied to the aforementioned porous element, it immediately flows, and thus the bone morphogenetic inductive factor is preferably homogeneously intermingled in the living body-absorbing member, which exhibits gradual release properties of the bone morphogenetic inductive factor, does not have living body damaging properties, and is gradually absorbed into cells in the living body, and at the same time gradually releases the bone morphogenetic inductive factor.

When the aforementioned living body-absorbing member is a porous element, the porous element itself has many communicated, open cavities, and so introduction of body fluids and cells is easy. Therefore, as rendering the living body-absorbing member to be a scaffold, on the surface thereof or the like is preferably made regenerated articular cartilage.

To this living body-absorbing member can, specifically, be applied a material as discussed above.

In addition, the aforementioned living body-absorbing member is preferably made in contact with an articular fluid in at least a portion thereof.

Furthemore, a ceramic porous element carrying a living body-absorbing member and a bone morphogenetic inductive factor on the inner surfaces is made buried on the articular face to fix articular cartilage on the articular face, thereby being able to regenerate the articular cartilage as well. According to such method, fixation of articular cartilage is made easy.

In this procedure, the aforementioned ceramic porous element is preferably buried in a position slightly deeper than the adjacent articular cartilage surface, that is, to be in a depressed state. Placing like this makes it possible to secure a space for regenerating articular cartilage and also to assimilate articular cartilage to be regenerated into the adjacent articular cartilage thereof.

With an articular fluid, itself or a specific component contained therein is thought to promote regenerate or form articular cartilage together with a bone morphogenetic inductive factor. In other words, an articular fluid contains a recovery-maintaining material for articular cartilage.

Accordingly, even in the case where a member containing a bone morphogenetic inductive factor as noted above and the gradual release thereof, or a ceramic porous element carrying a living body-absorbing member and a bone morphogenetic inductive factor on the inner surfaces is used, even though these are buried in a portion that is not made contact with an articular fluid or a specific component contained therein, articular cartilage is not formed.

As such, the aforementioned ceramic porous element is buried in an articular face, and then at least a portion of the ceramic porous element is preferably made contact with an articular fluid.

Articular cartilage regenerated as described above, to sufficiently have the function of a joint, is preferably homogeneously made grown to a thickness of 400 µm or more, more preferably to 500 µm or more.

In this manner, to obtain a sufficient amount of continuous articular cartilage, the aforementioned member for articular cartilage regeneration relating to the present invention can appropriately be used as a ceramic porous element carrying on the pore inner surface thereof a living body-absorbing member and a bone morphogenetic inductive factor.

Moreover, as discussed above, the use of a member for articular cartilage regeneration relating to the present invention, even without employing a special factor such as a bone morphogenetic inductive factor, if the member is a specific porous element comprising hydroxyapatite allowed to have already been applied to the human body, enables early regeneration of articular cartilage in the original thickness.

According to a method relating to the present invention as described above, for instance, for a patientwho suffers damage of articular cartilage over a wide area due to violent exercise or the like, collected self articular cartilage of the side portion of the articular face that is not so important in the function of articular cartilage is implanted in the affected part. Then, surgery is also possible in which in the perforation of the side portion arising from this procedure is buried a member for regeneration of articular cartilage relating to the present invention to compensate the collected articular cartilage.

Also, through the use of a member for articular cartilage regeneration relating to the present invention, in vitro an environment similar to the articular portion in vivo is formed, and in the environment thereof articular cartilage is cultivated utilizing articular cells and mesenchymal cells, mesenchymal stem cells, bone marrow cells, or the like, collected from a patient to be capable of implant it in an affected part as well. In this manner, a treatment method of replacing the conventional artificial articular treatment, which is large in burden and pain to a patient is possible to be established as well.

Next, a cultivating method of articular cartilage relating to the present invention will be set forth. A cultivating method of articular cartilage relating to the present invention is characterized by placing a living body-absorbing member of a porous element, which contains a bone morphogenetic inductive factor, and which has gradual release properties thereof, close to or near cells to be possibly articular cartilage, and contacting at least a portion of the aforementioned member with the articular fluid to cultivate the articular cartilage.

Such method enables the cultivation of articular cartilage in vitro using a self articular fluid. Although the implantation of articular cartilage itself cultivated in vitro is said to be difficult, a making method of a state in which a bone similarly cultivated in vitro to be integrated therewith, or of placing indifferent cells around articular cartilage cultivated in vitro, or a similar method makes it possible to implant articular cartilage cultivated in vitro as well.

In addition, a ceramic porous element carrying on the pore inner surface thereof a living body-absorbing member and a bone morphogenetic inductive factor is placed close to or near cells to be possibly articular cartilage, or into cavities of a ceramic porous element is introduced cells to be possibly articular cartilage, and then the aforementioned ceramic porous element is made contact with an articular fluid in at least a portion thereof to thereby be capable of cultivating articular cartilage as well.

In this manner, placing cells to be possibly articular cartilage and thus indifferent cells around articular cartilage to be cultivatedmakes it possible to implant articular cartilage cultivated in vitro.

Here, as long as a cell to be possibly articular cartilage comprises a mesenchymal stem cell, an articular cell, a cell forming articular cartilage by gene recombination, or the like, the cell to be possibly articular cartilage is not particularly limited, but a mesenchymal stem cell is more preferable.

Additionally, in the above-described cultivation methods, when at least a portion of the aforementioned member is made contact with an articular fluid, to be rendered similar to the environment of the articular space inside, the articular fluid may be pressure applied.

Furthermore, on at least a portion of the pore inner surface of a ceramic porous element carrying on the pore inner surface thereof a living body-absorbing member and a bone morphogenetic inductive factor is formed a bone, and then further on at least a portion of the surface thereof can also be cultivated articular cartilage by means of a cultivation method as described above.

In this way, directly below the articular cartilage thus cultivated, forming a similarly cultivated bone in an integrated state also permits the implantation of the articular cartilage cultivated in vitro.

For articular cartilage cultivated as discussed above, to sufficiently carry the function of a joint, the thickness is homogeneous and preferably 400 µm or more, more preferably 500 µm or more.

Thus, to obtain a sufficient amount of continuous articular cartilage, the aforementioned member for articular cartilage regeneration relating to the present invention can appropriately be used as a ceramic porous element carrying on the pore inner surface thereof a living body-absorbing member and a bone morphogenetic inductive factor.

Next, an artificial articular cartilage for implantation relating to the present invention will be set forth. An artificial articular cartilage for implantation relating to the present invention is characterized in that on at least a portion of the pore inner surface of a ceramic porous element carrying on the pore inner surface thereof a living body-absorbing member and a bone morphogenetic inductive factor, an articular cartilage is formed.

Such artificial articular cartilage for implantation can be obtained by a cultivating method of articular cartilage as discussed above.

In addition, for the aforementioned artificial articular cartilage for implantation, on at least a portion of the surface of the bone formed by fixation of bone cells in the pore of the aforementioned ceramic porous element, an articular cartilage layer is formed, or on at least a portion of the surface of the bone formed by fixation of bone cells in the pore of the aforementioned ceramic porous element, a bone cell layer not containing the ceramic porous element is formed, and further on at least a portion of the surface of the bone cell layer, the formation of an articular cartilage layer is preferable.

As described above, implanting only articular cartilage within the body is difficult from the viewpoint of adhesion properties to a bone and the like, and therefore in artificial articular cartilage for implantation, as discussed above, the articular cartilage layer is preferably integrated with the bone of the under surface thereof or the bone cells.

In this procedure, as the aforementioned ceramic porous element carrying on the pore inner surface thereof a living body-absorbing member and a bone morphogenetic inductive factor, the member for articular cartilage regeneration as mentioned above, relating to the present invention, can be suitably used.

Furthermore, a regeneration or formation method of or a cultivation method of articular cartilage relating to the present invention is characterized in that close to or near the articular cartilage collected from articular cartilage within the living body or from the living body, a bone morphogenetic inductive factor is gradually released. An articular cartilage layer is preferably integratedly formed with an existent living body articular cartilage, and also activating articular cells of the living body makes it possible to promote the regeneration or formation or cultivation of articular cartilage.

In this case, the aforementioned bone morphogenetic inductive factor is preferably gradually released in the presence of an articular fluid.

Hereinafter, the present invention will be set forth in terms of Examples in detail; however, the invention is by no means limited by the Examples below.

### [Example 1]

100 Milligrams of PLA-DX-PEG (PLA:DX:PEG=45:17:38 (molar ratio)) comprising a copolymer having a number average molecular weight of 9300 comprising DL-lactide, p-dioxanone and polyethylene glycol, and 20 µg of rhBMP-2 were blended and the resultant material was diluted with acetone to prepare a gel-like mixture.

This gel-like mixture was infiltrated into a porous element of hydroxyapatite (diameter 4 mm, length 4 mm, porosity 75%, pore diameter 200 µm) and the resulting material was allowed to stand for a while. Subsequently, the acetone was evaporated to form a homogeneous mixture layer of a living body-absorbing member and a bone morphogenetic inductive factor on the pore inner surface of the apatite porous element, thereby obtaining a member for articular cartilage regeneration.

The member for articular cartilage regeneration thus obtained was buried on the articular face of the femur perforated in the size thereof of a rabbit, and then the joint was put back to the origin and further the open portion was sutured.

After 3, 6, and 12 weeks, the buried portion of the aforementioned member was observed from the surface side.

After 3 weeks, the regeneration of the articular cartilage was slight, but after 6 weeks, it was visually regenerated to the original shape. Furthermore, after 12 weeks, the articular cartilage was completely regenerated.

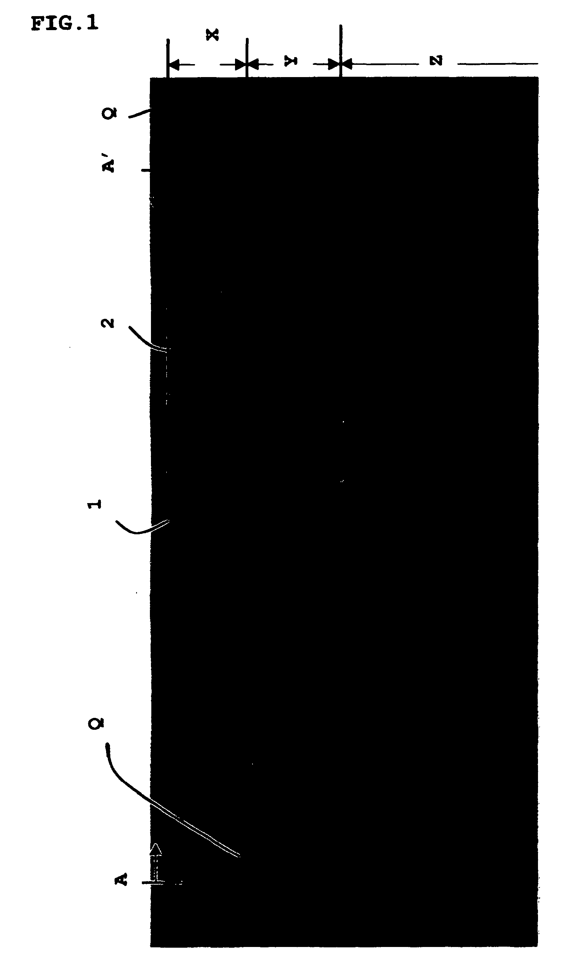

Microscope photographs of the buried portion after 6 weeks are shown in terms of pictures in Figs. 1 to 3.

In Fig. 1, the interval taken along A-A' is a portion in which the member for articular cartilage regeneration has been buried. In the lower layer of an articular surface 1 is formed the layer X of a regenerated articular cartilage 2. Also, between the aforementioned regenerated articular cartilage layer X and the ceramic porous element layer Z, the layer Y only comprising a bone, and without containing a ceramic porous element is present.

Additionally, it was confirmed that into the pore of the ceramic porous element are incorporated bone cells and that the bone is regenerated.

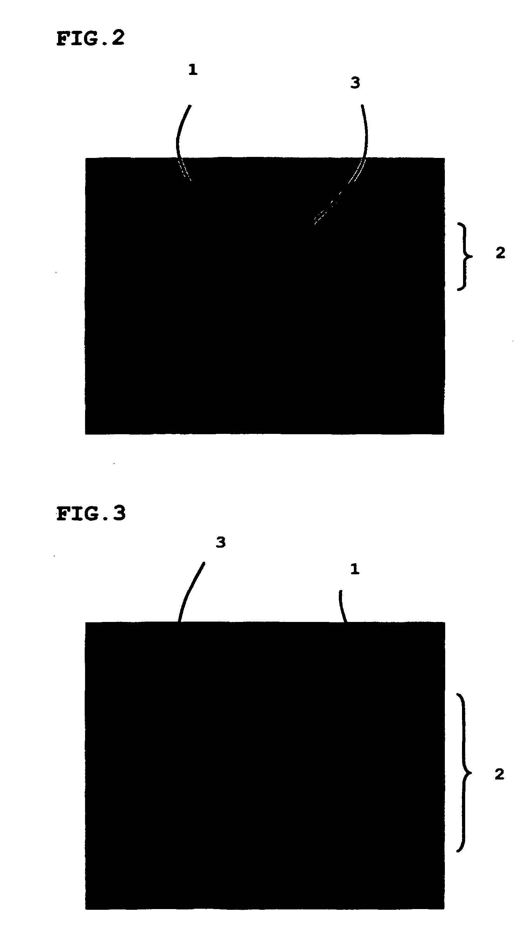

Fig. 2 shows a portion of an enlarged photograph taken along the line A-A' in Fig. 1, and Fig. 3 further shows an enlarged photograph of an upper portion layer thereof.

As shown in Fig. 1, it is confirmed that after 6 weeks the regenerated articular cartilage is formed about 500 µm in thickness (about 2/3 of a normal thickness of articular cartilage of a rabbit).

Moreover, in the portion of the line taken along A and A' of Figs. 1 to 3, the aforementioned regenerated articular cartilage 2 is continuously formed to the extent that it cannot be distinguished from the border of the original articular cartilage, the regenerated articular cartilage layer X (Q portion) of the vicinity thereof is recognized as being particularly formed into a thick layer.

Furthermore, as shown in Fig. 3, in the regenerated articular cartilage layer 2, many articular cartilage cells 3 are confirmed.

Also, after 12 weeks, the buried portion (the interval taken along the line A-A') is recovered to the extent of not being distinguished.

### [Comparative Example 1]

A member for articular cartilage regeneration was produced as in Example 1 with the exception that the member did not contain a bone morphogenetic inductive factor.

The member for articular cartilage regeneration thus obtained was buried, as in Example 1, in a femur articular face of a rabbit, and after 3, 6, and 12 weeks, the buried portion was observed from the surface side.

In the case after 3, 6, and 12 weeks, although a bone is formed, the regeneration of articular cartilage was not observed.

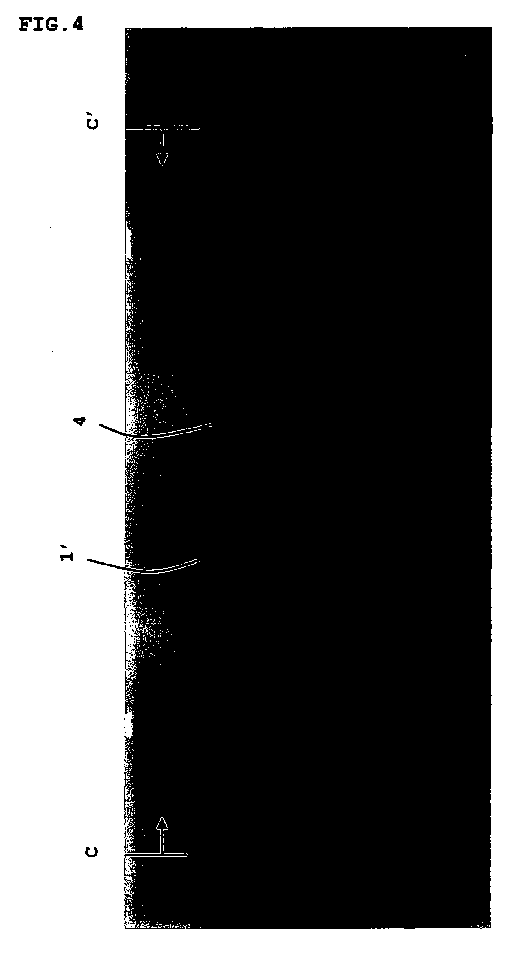

Microscope photographs of the buried portion after 6 weeks are shown in terms of pictures in Figs. 4 to 6. In Fig. 4, the interval taken along the line C-C' is a portion in which the member for articular cartilage regeneration has been buried.

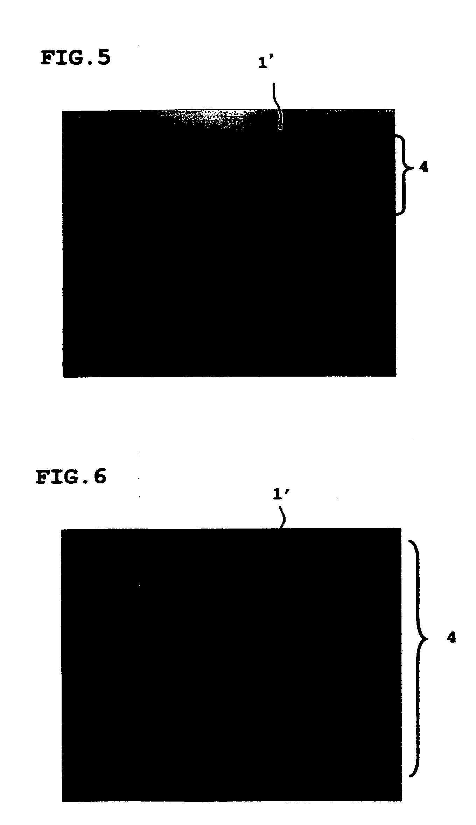

Fig. 5 shows an enlarged photograph of a portion of the interval taken along the line C-C' of Fig. 4, and Fig. 6 further shows an enlarged photograph of an upper portion thereof.

As indicated in Fig. 4, even after 6 weeks, the border portion of the buried portion (interval taken along the line C-C') and the original articular cartilage is clearly recognized particularly in the C' portion.

In addition, as shown in Fig. 6, although a fibrous articular cartilage 4 is confirmed in the buried portion (interval taken along the line C-C' ) of the aforementioned member, the formation of articular cartilage is not recognized.

Additionally, the fibrous cartilage 4, in which two-type collagen was not recognized, was confirmed as not being articular cartilage.

Furthermore, even after 12 weeks as well, cartilage was not formed.

### [Comparative Example 2]

A member for articular cartilage regeneration produced as in Example 1 was buried in a femur side face of a rabbit, and after 3, 6, and 12 weeks, the buried portion was observed.

As a result, after 6 weeks, the regeneration of a bone was recognized, but cartilage was not formed the surface thereof.

As discussed above, the case using a member of articular cartilage regeneration in which a bone morphogenetic inductive factor relating to the present invention was carried (Example 1) was recognized as continuously forming articular cartilage in a sufficient amount, in contrast to the case in which a bone morphogenetic inductive factor was not carried (Comparative Example 1).

In addition, the case where a member of articular cartilage regeneration in which a bone morphogenetic inductive factor was carried was made contact with an articular fluid (Example 1) formed articular cartilage, but the case not making contact with an articular fluid (Comparative Example 2) was recognized as not forming articular cartilage.

### [Example 2]

A porous element of hydroxyapatite, having a porosity of 75% and a cavity diameter of 200 µm, was produced to thereby prepare a cylindrical element having a diameter of 4 mm and a length of 4 mm.

A knee joint of a rabbit was cut open, and a perforation which had a diameter of 4 mm and a portion of which reached the depth of the position of a growth cartilage layer 3 was made in the femur articular face, and into the perforation, the resulting porous element was buried such that the surface thereof was 1 mm deeper than the articular cartilage layer, i.e., d= 1 mm in Fig. 11. Subsequently, the joint was put back to the origin and further the open portion was sutured to make a movable state.

The above-described procedure was carried out for 5 rabbits (n = 5).

### [Example 3]

A porous element of hydroxyapatite as in Example 2 was produced.

A knee joint of a rabbit was treated as in Example 2 with the exception that in the perforated depth of this porous element, the surface thereof was 2 mm deeper than the articular cartilage layer, i.e., d= 2 mm in Fig. 11.

### [Comparative Example 3]

A porous element of hydroxyapatite as in Example 2 was produced.

A knee joint of a rabbit was treated as in Example 2 with the exception that in the perforated depth of this porous element, the surface thereof was equal to the depth of the under surface of the articular cartilage layer, i.e., d= 0 in Fig. 11.

### [Comparative Example 4]

A knee joint of a rabbit was treated as in Example 2 with the exception that in the perforated depth of this porous element, the surface thereof was 0.5 mm shallower than the depth of the under surface of the articular cartilage layer.

### [Comparative Example 5]

As a control test, a knee joint of a rabbit was cut open, and a perforation having a diameter of 4 mm and a depth of 4 mm was made in the femur articular face, and nothing was placed into the perforation. Subsequently, the joint was put back to the origin and further the open portion was sutured to make a movable state.

The above-described treatment was carried out for 5 rabbits (n= 5).

After 3 weeks, in the above-described Examples 2 and 3 and Comparative Examples 3 to 5, each of the treated portions was observed.

As a result, for the cases where a porous element was buried in a position deeper than the under surface of the articular cartilage layer (Examples 2 and 3), regardless of a short period of time of 3 weeks, an articular inferior bone was formed on the upper surface of the porous element, and further on the surface thereof, with a thickness equivalent to that of adjacent, surrounding, healthy articular cartilage, about 60% thereof was recognized as articular cartilage being completely regenerated.

With this regenerated articular cartilage, the surface of the border portion was continuously smoothly formed.

In addition, in the under surface of the border portion of the regenerated articular cartilage and adjacent, surrounding, healthy articular cartilage, some articular cartilage was recognized as being slightly thickly formed.

On the other hand, in the cases where a porous element was buried in the depth equivalent to the depth position of the under surface of the articular cartilage layer, or shallower than the depth position (Comparative Examples 3 and 4), articular cartilage was not regenerated at all.

This suggests that where there is not a space between articular cartilage and a porous element, articular cartilage is not regenerated.

Also, as a control test, in the case where no porous was buried (Comparative Example 5), articular cartilage was not regenerated at all.

### [Example 4]

100 Milligrams of PLA-DX-PEG (PLA:DX:PEG= 45:17:38 (molar ratio)) comprising a copolymer having a number average molecular weight of 9300 comprising DL-lactide, p-dioxanone and polyethylene glycol, and 20 µg of rhBMP-2 were blended and the resultant material was diluted with acetone to prepare a gel-like mixture.

This gel-like mixture was infiltrated into a porous element of hydroxyapatite prepared as in Example 2 and the resulting material was allowed to stand for a while. Subsequently, the acetone was evaporated to coat the pore inner surface of the apatite porous element with a homogeneous mixture layer of a homogeneous mixture layer of a living body-absorbing member and a bone morphogenetic inductive factor, thereby obtaining a member for articular cartilage regeneration.

Through the use of this member for articular cartilage regeneration, as in Example 2, a knee joint of a rabbit was treated.

When after 3 weeks the treated portion was observed, for the case using a member for articular cartilage regeneration comprising a porous element coated with both a living body-absorbing member and a bone morphogenetic inductive factor (Example 4), portions in which the articular cartilage is regenerated in almost complete state were about 100%, the certainty of regeneration of the articular cartilage was recognized as being more excellent as compared with the members for regeneration of articular cartilage only comprising a porous element (Examples 2 and 3). In particular, the homogeneity of thickness of the whole was recognized as being excellent.

### [Example 5]

A porous element of hydroxyapatite as in Example 2 was produced.

A knee joint of a rabbit was treated as in Example 2 with the exception that a knee joint of a rabbit was cut open in the femur articular face, and that a perforation which had a diameter of 4 mm and which did not reach the depth of the position of a growth cartilage layer was made in the femur articular face.

Additionally, the length of the aforementioned porous element was, as appropriate, finely adjusted to be accommodated within the aforementioned perforation.

### [Example 6]

A porous element of hydroxyapatite as in Example 2 was produced.

A knee joint of a rabbit was treated as in Example 2 with the exception that a knee joint of a rabbit was cut open in the femur articular face, and that a perforation which had a diameter of 4 mm and which reached the depth of the position of a growth cartilage layer was made in the femur articular face.

In addition, the deep portion of the aforementioned perforation was made in an empty state.

When after 3 weeks the treated portion was observed, for both the case of perforating until the depth position not reaching the growth cartilage layer (Example 5) and the case of perforating until reaching the proximity of the bone marrow penetrating the growth cartilage layer (Example 6), it was confirmed that a cartilage inferior bone was formed on the upper surface of the porous element, and further that on the surface thereof articular cartilage was regenerated.

However, with the case where perforation was carried out until the depth position not reaching the growth cartilage layer (Example 4), regeneration of articular cartilage that is said to be almost complete was about 20%. Of these, some are inhomogeneous in the thickness of regenerated articular cartilage, and needed slightly more time until sufficient regeneration.

On the other hand, for the case where perforation was conducted until penetrating the growth cartilage layer and reaching the proximity of the bone marrow (Example 6), as in the case where a portion of the perforation reached the depth position reaching the growth cartilage layer (Example 2), it was confirmed that about 60% of articular cartilage was almost completely regenerated.

### Industrial Applicability

Use of a member for articular cartilage regeneration relating to the present invention makes it possible to regenerate articular cartilage in a nearly natural state, and to form articular cartilage with a pract ical level.

In addition, according to a regeneration method of and a cultivation method of articular cartilage relating to the present invention, the only use of a member comprising material allowed to have already been applied to the human body, in an environment near nature, can integrate the articular cartilage with adjacent, surrounding, existent articular cartilage under good conditions and, under continuous conditions, early regenerate the articular cartilage having original thickness.

Furthermore, the use of artificial articular cartilage for implantation obtained by these methods, relating to the present invention, raises the possibility of contributing to the establishment of a treatment method replacing the conventional artificial cartilage treatment, which is large in burden and pain to a patient.

## Claims

1. A member for articular cartilage regeneration is **characterized in that**
the member comprises a hydroxyapatite porous element having a number of pores distributed therein, substantially all of said pores being three-dimensionally communicated to each other through open portions, a porosity of from 50% to 90%, both inclusive, and an average pore diameter of from 100 µm to 600 µm, both inclusive.

2. A member for articular cartilage regeneration according to claim 1, **characterized in that**
the pores of the porous element are formed by agitation foaming, and the pores which is almost sphere-like, adjacent pores are opened to each other in contact portions to form continuous sphere-like open pores.

3. A member for articular cartilage regeneration according to claim 1, **characterized in that**
the average diameter of the open portion of each pore of the porous element is 20 µm or more.

4. A member for articular cartilage regeneration which comprises a ceramic porous element; and a living body-absorbing member;
said ceramic porous element being formed with pores, substantially all of said pores cooperating to form three-dimensionally communicated continuous sphere-like open pores by way of openings thereof to such an extent that, of said pores, ones with a pore diameter of 5µm or more occupying 85% or more of the total pore volume in terms of a pore diameter distribution determined by mercury porosimeter measurement, said living body-absorbing member being carried on a pore inner surface of said ceramic porous element and containing a bone morphogenetic inductive factor therewithin.

5. A member for articular cartilage regeneration according to claim 4, **characterized in that**
the bone morphogenetic inductive factor is any one selecting from the group consisting of bone morphogentic protein (BMP), transforming growth factor β (TGF-β ) , an osteoinductive factor (OIF), an insulin-like derived growth factor (IGF), a platlet derived growth factor (PDGF), and a fibroblast growth factor (FGF).

6. A member for articular cartilage regeneration according to claim 5, **characterized in that**
the bone morphogenetic inductive factor comprises recombinant human bone morphogentic protein (rhBMP).

7. A member for articular cartilage regeneration according to claim 4, **characterized in that**
the bone morphogenetic inductive factor is homogeneously intermingled in the living body-absorbing member.

8. A member for articular cartilage regeneration according to claim 4, **characterized in that**
the pore of the porous ceramic element is formed by agitation foaming, has a porosity of 50% to 90%, both inclusive, an average pore diameter of 100 µm to 600 µm, both inclusive, and an average diameter of the open portion of each pore in the sphere-like open pores being 20 µm, or more.

9. A member for articular cartilage regeneration according to claim 4, **characterized in that**
the ceramic porous element comprises at least one kind selecting from the group consisting of alumina, zirconia, silica, mullite, deopside, wollastnite, alite, belite, arkelmanite, monticellite, glass for the living body, and calcium phosphate-based ceramics.

10. A member for articular cartilage regeneration according to claim 9, **characterized in that**
the calcium phosphate-based ceramics comprises at least one species selected from the group consisting of hydroxyapatite, tricalcium phosphate, and apatite fluoride.

11. A member for articular cartilage regeneration according to claim 10, **characterized in that**
the calcium phosphate-based ceramics comprises hydroxyapatite.

12. A member for articular cartilage regeneration according to claim 4, **characterized in that**
the living body-absorbing member has the gradual release properties of a bone morphogenetic inductive factor.

13. A member for articular cartilage regeneration according to claim 4, **characterized in that**
the living body-absorbing member comprises an organic compound.

14. A member for articular cartilage regeneration according to claim 4, **characterized in that**
the living body-absorbing member comprises at least one species selecting from the group consisting of a polymer of lactic acid and/or glycolic acid, a block copolymer of a polymer of lactic acid and/or glycolic acid and polyethylene glycol, a copolymer of lactic acid and/or glycolic acid, p-dioxanone and polyethylene glycol, and atherocollagen.

15. A member for articular cartilage regeneration according to claim 4, **characterized in that**
the living body-absorbing member comprises a block copolymer of polylactic acid and polyethylene glycol.

16. A member for articular cartilage regeneration according to claim 15, **characterized in that**
in the block copolymer of polylactic acid and polyethylene glycol, polylactic acid has a number average molecular weight of 400 to 1000000 and the molar ratio of the polylactic acid to the polyethylene glycol ranges from 25:75 to 75:25.

17. A member for articular cartilage regeneration according to claim 4, **characterized in that**
the living body-absorbing member comprises a copolymer of lactic acid and/or glycolic acid, p-dioxanone and polyethylene glycol.

18. A producing method ofa member for articular cartilage regeneration, **characterized by** comprising:
a step of adding a living body-absorbing material to a solvent or a dispersing medium and then blending a bone morphogenetic inductive factor therewith to prepare a mixture solution;
a step of infiltrating the mixture solution into a ceramic porous element in which the pores are formed by agitation foaming and which forms a continuous, sphere-like, open pore produced by three-dimensionally communicating each pore to each other through open portions; and
a step of removing the solvent or the dispersing medium in the the porous ceramic element and carrying a living body-absorbing member and a bone morphogenetic inductive factor to obtain the member for articular cartilage regeneration.

19. A producing method of a member for articular cartilage regeneration according to claim 18, **characterized in that**
in the step of preparing the mixture solution, the living body-absorbing member comprises an organic compound, and acetone is used as the solvent or the dispersing medium.

20. A regenerating method of articular cartilage, **characterized in that**
a porous element is made buried in a position deeper than the under surface of the articular cartilage of a articular face.

21. A regenerating method of articular cartilage according to claim 20, **characterized in that**
the porous element is buried in such a way that at least a portion of the porous element is made contact with mesenchymal cells, mesenchymal stem cells, or bone marrow cells, in a bone.

22. A regenerating method ofarticular cartilage according to claim 20, **characterized in that**
the porous element is buried such that the upper surface of the porous element is exposed to the articular face.

23. A regenerating method of articular cartilage according to claim 20, **characterized in that**
after the porous element is buried, at least a portion of the porous element is made contact with the articular fluid.

24. A regenerating method of articular cartilage, **characterized by** comprising:
cutting an articular capsule open to expose the articular face,
making perforation in a desired position, embeding a porous element in a site deeper than the under surface of the articular cartilage layer within the perforation, and subsequently
suturing the articular capsule.

25. A regenerating method of articular cartilage according to claim 24, **characterized in that**
the perforation is formed so that the lower end thereof reaches the proximity to the bone marrow.

26. A regenerating method of articular cartilage according to claim 20 or 24, **characterized in that**
as the porous element, a porous element, the porosity of which is from 50% to 90%, both inclusive, having as a whole a communicated pore capable of permeation and movement by cells is used.

27. A regenerating method of articular cartilage according to claim 20 or 24, **characterized in that**
as the porous element, a porous element comprising hydroxyapatite is used.

28. A regenerating method of articular cartilage according to claim 20 or 24, **characterized in that**
as the porous element, a porous element, the pores of which are formed by agitation foaming is used.

29. A regenerating method of articular cartilage, **characterized by**:
placing on the articular face a living body-absorbing member of a porous element, which contains a bone morphogenetic inductive factor, and which has gradual release properties thereof, and fixing.

30. A regenerating method of articular cartilage according to claim 29, **characterized in that**
at least a portion of the living body-absorbing member is made contact with the articular fluid.

31. A regenerating method of articular cartilage, **characterized in that**
a ceramic porous element carrying on the pore inner surface thereof a living body-absorbing member and a bone morphogenetic inductive factor is made buried in an articular face to regenerate articular cartilage in the articular face.

32. A regenerating method of articular cartilage according to claim 31, **characterized in that**
after the ceramic porous element is buried in the articular face, at least a portion of the ceramic porous element is made contact with the articular fluid.

33. A regenerating method of articular cartilage according to claim 31, **characterized in that**
as the ceramic porous element carrying on the pore inner surface thereof a living body-absorbing member and a bone morphogenetic inductive factor, a member for articular cartilage regeneration according to claim 4 is used.

34. A regenerating method of articular cartilage according to claim 29 or 31, **characterized in that**
the articular cartilage is made grown in such a way that the articular cartilage has a homogeneous thickness of 400 µm or more.

35. A cultivating method of articular cartilage, **characterized by**:
placing a living body-absorbing member of a porous element, which contains a bone morphogenetic inductive factor, and which has gradual release properties thereof, close to or near cells to be possibly articular cartilage, and
contacting at least a portion of the member with the articular fluid.

36. A cultivating method of articular cartilage, **characterized by**:
placing a ceramic porous element carrying on the pore inner surface thereof a living body-absorbing member and a bone morphogenetic inductive factor close to or near cells to be possibly articular cartilage, and
contacting at least a portion of the ceramic porous element with the articular fluid.

37. A cultivating method of articular cartilage, **characterized by**:
incorporating cells to be possibly articular cartilage into the pore inside of a ceramic porous element carrying on the pore inner surface thereof a living body-absorbing member and a bone morphogenetic inductive factor, and
contacting at least a portion of the ceramic porous element with the articular fluid.

38. A cultivating method of articular cartilage according to any one of claims 35 to 37, **characterized in that**
the cell to be possibly articular cartilage comprises a mesenchymal stem cell.

39. A cultivating method of articular cartilage according to claim 36 or 37, **characterized in that**
on at least a portion of the pore inner surface of a ceramic porous element carrying on the pore inner surface thereof a living body-absorbing member and a bone morphogenetic inductive factor is formed a bone, and **characterized in that**
then further on at least a portion of the surface of the ceramic porous element, articular cartilage is cultivated.

40. A cultivating method of articular cartilage according to claim 36 or 37, **characterized in that**
for the ceramic porous element carrying on the pore inner surface thereof a living body-absorbing member and a bone morphogenetic inductive factor is used a member for articular cartilage regeneration according to claim 4.

41. A cultivating method of articular cartilage according to any one of claims 39, 41 and 42, **characterized in that**
the articular cartilage is made grown in such a way that the articular cartilage has a homogeneous thickness of 400 µm or more.

42. An artificial articular cartilage for implantation, **characterized in that**
on at least a portion of the surface of a ceramic porous element carrying on the pore inner surface thereof a living body-absorbing member and a bone morphogenetic inductive factor is formed articular cartilage.

43. An artificial articular cartilage for implantation according to claim 42, **characterized in that**
the articular cartilage has a homogeneous thickness of 400 µm or more.

44. An artificial articular cartilage for implantation according to claim 42, **characterized in that**
on at least a portion of the surface of a bone formed by fixation of the bone cells in the pore inside of the ceramic porous element is formed an articular cartilage layer.

45. An artificial articular cartilage for implantation according to claim 42, **characterized in that**
on at least a portion of the surface of a bone formed by fixation of the bone cells in the pore inside of the ceramic porous element is formed a bone cell layer not containing a ceramic porous element, and **characterized in that**
further, on at least a portion of the surface of the bone cell layer is formed an articular cartilage layer.

46. An artificial articular cartilage for implantation according to claim 42, **characterized in that**
the ceramic porous element carrying on the pore inner surface thereof a living body-absorbing member and a bone morphogenetic inductive factor comprises a member for articular cartilage regeneration according to claim 4.

47. A regeneration method of or a cultivation method of articular cartilage, **characterized in that**
close to or near the articular cartilage collected from articular cartilage within the living body or from the living body is gradually released a bone morphogenetic inductive factor.

48. A regeneration method of or a cultivation method of articular cartilage according to claim 47, **characterized in that**
the bone morphogenetic inductive factor is gradually released in the presence of an articular fluid.
